# EUROPÄISCHE PATENTSCHRIFT

(11) **EP 2 015 816 B1**
(45) Veröffentlichungstag und Bekanntmachung des Hinweises auf die Patenterteilung: **20.06.2012**
(21) Anmeldenummer: 06753565.8
(22) Anmeldetag: 11.05.2006
(51) Int. Cl.: A61M 25/00

(54) **KATHETEREINRICHTUNG FÜR PERKUTANE EINGRIFFE**
CATHETER DEVICE FOR PERCUTANEOUS INTERVENTIONS
DISPOSITIF DE CATHÉTÉRISME POUR INTERVENTIONS PERCUTANÉES

(43) Veröffentlichungstag der Anmeldung: 21.01.2009
(73) Patentinhaber: Krombach, Gabriele, 52070, Aachen (DE)
(72) Erfinder: BÜCKER, Arno, 52349 Düren (DE); PFEFFER, Joachim, G., 52070 Aachen (DE); FRIEBE, Michael, 45657 Recklinghausen (DE); KROMBACH, Gabriele, 52070 Aachen (DE)
(74) Vertreter: Gesthuysen, von Rohr & Eggert
(86) Internationale Anmeldenummer: PCT/EP2006/004429
(87) Internationale Veröffentlichungsnummer: WO 2007/131516

(56) Entgegenhaltungen:
- WO-A-99/49773
- US-A- 4 222 380
- US-A1- 2004 199 141

## Beschreibung

Die Erfindung betrifft eine Kathetereinrichtung für perkutane Eingriffe, insbesondere für Injektionen, Biopsien oder dergleichen. Eine solche Kathetereinrichtung ist aus WO9949773 bekannt.

Die Fortschritte in der Molekularbiologie und Gentechnologie der letzten Jahre haben zur Entwicklung verschiedenartiger therapeutischer Substanzen geführt, die zum Beispiel die Angioneogenese im ischämisch geschädigten Myokard oder die Ansiedlung von omnipotenten Stammzellen und Differenzierung zu Myozyten in Myokardnarben anstreben. Klinische Untersuchungen bei Patienten zeigten, daß therapeutische Gewebekonzentrationen derartiger Substanzen nur nach direkter Injektion in das Gewebe erzielt werden konnten. Dabei läßt sich eine Injektion derartiger Substanzen ohne weiteres bei einer Operation am offenen Herzen durchführen. Derartige Operationen sind jedoch stets mit einem nicht unerheblichen Aufwand und einem entsprechenden Risiko verbunden. Einfacher erscheint in diesem Zusammenhang die perkutane Injektion mittels einer Kathetereinrichtung. Problematisch ist hierbei jedoch, daß bei einer perkutanen Injektion am Katheter eine entsprechende Nadel vorgesehen werden muß und beim Einbringen des Katheters in den Körper beispielsweise an einer Leiste oder einer Armbeuge bis in das Herz das Vorführen durch die Arterien nicht zu einer Verletzung der Gefäße führen darf. Ein weiteres Problem bei der perkutanen Injektion ergibt sich dadurch, daß es unbeabsichtigt passieren kann, daß das Herzgewebe bei der Injektion mit durchstochen wird. Das ungewollte Durchstechen des Herzgewebes birgt die Gefahr des Vollaufens des Herzbeutels mit Blut und folgender Kompression des Herzens mit möglicher Todesfolge in sich.

Ähnliche Probleme ergeben sich beispielsweise bei perkutanen Gewebeentnahmen, bei denen eine Biopsiezange mittels eines Katheters an die Entnahmestelle gebracht wird.

Aufgabe der vorliegenden Erfindung ist es nun, eine Kathetereinrichtung für perkutane Eingriffe wie weitar in Anspruch 1 offengelest zu Verfügung zu stellen, bei der die vorgenannten Nachteile vermieden werden.

Zur Lösung der vorgenannten Aufgabe ist eine Kathetereinrichtung für perkutane Eingriffe, insbesondere für Injektionen, Biopsien oder dergleichen vorgesehen, die einen Außenkatheter und ein Werkzeug für den Eingriff aufweist. Bei dem Werkzeug kann es sich beispielsweise um eine Injektionsnadel, eine Biopsiezange oder aber um Elektroden handeln. Die vorgenannte Aufzählung ist jedoch nicht abschließend, so daß auch andere Werkzeuge erfaßt sein können. Bei der erfindungsgemäßen Kathetereinrichtung ist das Werkzeug am proximalen Ende der Kathetereinrichtung vorgesehen. Wesentlich ist nun, daß das Werkzeug in einem Zuführzustand im Außenkatheter aufgenommen ist. Dabei befindet sich die Werkzeugspitze des Werkzeugs vollständig im Außenkatheter, das heißt die Werkzeugspitze ist darin versenkt, also gegenüber dem Außenkatheter zurückversetzt oder aber mit dem proximalen Ende des Außenkatheters ausgefluchtet und keinesfalls überstehend. Der Einführzustand bezeichnet dabei den Zustand, bei dem der Außenkatheter zusammen mit dem Werkzeug in den Körper eingeführt und bis an die Eingriffsstelle geschoben wird. Ist die Eingriffsstelle erreicht, befindet sich die Kathetereinrichtung im Eingriffszustand. Wichtig bei der Erfindung ist nun, daß das Werkzeug zum Eingriff relativ zum Außenkatheter bewegbar ist, und zwar so weit, daß zumindest die Werkzeugspitze im Eingriffszustand über das proximale Ende des Außenkatheters übersteht. Die erfindungsgemäße Ausgestaltung ermöglicht es im Ergebnis, das Werkzeug aus einem versenkten Zustand zum Eingriff an der Eingriffstelle auf eine vordefinierte Länge auszufahren und dieses bei Bedarf nach dem Eingriff wieder in den Außenkatheter einzufahren, um beim Abziehen des Katheters die Gefahr einer Verletzung der Gefäße zu vermeiden.

Bei einer bevorzugten Ausführungsform der vorliegenden Erfindung ist zum Ausfahren des Werkzeugs aus dem Außenkatheter ein innerhalb des Außenkatheters befindlicher Innenkatheter vorgesehen, der relativ zum Außenkatheter bewegbar ist. Dabei ist das Werkzeug am proximalen Ende des Innenkatheters vorgesehen, so daß das Werkzeug an der Eingriffstelle aus dem Außenkatheter ausgefahren werden kann. Am distalen Ende der Kathetereinrichtung steht der Innenkatheter über den Außenkatheter über, so daß während des Eingriffs über entsprechende Mittel der Innenkatheter bewegt werden kann.

Bei Verwendung eines Innenkatheters ist es zweckmäßig, das Werkzeug mit seinem distalen Ende am Innenkatheter zu befestigen. Alternativ kann das Werkzeug jedenfalls bei verschiedenen Ausführungsformen auch einstückig mit dem Innenkatheter ausgebildet sein.

Bei einer anderen Ausführungsform ohne Innenkatheter ist vorgesehen, daß zum Ausfahren des Werkzeugs am proximalen Ende des Außenkatheters eine Vorschubeinrichtung befestigt ist, wobei das Werkzeug wiederum an der Vorschubeinrichtung befestigt ist. Bei einer bevorzugten Ausgestaltung dieser Ausführungsform weist die Vorschubeinrichtung wenigstens eine Feder auf. Hierbei kann es sich beispielsweise um eine Schraubenfeder handeln, die im Zufiihrzustand komprimiert ist. Um das Werkzeug aus dem eingefahrenen, versenkten Zustand ausfahren zu können, ist bei dieser Ausführungsform wenigstens ein Auslösemittel zur Freigabe der Feder vorgesehen. Zweckmäßigerweise handelt es sich hierbei um einen von außen betätigbaren Auslösedraht. Im übrigen kann bei dieser Ausführungsform eine Feststellung der Vorschubeinrichtung sowohl im eingefahrenen Zustand als auch im ausgefahrenen Zustand vorgesehen sein, um ein unbeabsichtigtes Aus- oder Einfahren des Werkzeugs zu vermeiden.

Gerade bei perkutanen Eingriffen am Herzen kann es dazu kommen, daß bei Injektionen die Nadel zu tief in das Herzgewebe eingestochen oder das Herzgewebe gar durchstochen wird. Zur Beseitigung dieses Problems ist am Werkzeug und/oder am Innenkatheter ein Anschlag zur Eingriffstiefenbegrenzung des Werkzeugs beim Eingriff vorgesehen. Ein derartiger Anschlag stellt sicher, daß beispielsweise eine Nadel nur über eine genau vorgegebene Länge in das betreffende Gewebe eindringen kann.

Ein Anschlag der vorgenannten Art läßt sich auf vielerlei Weise realisieren. Um in jedem Falle eine Eingriffs- bzw. Einstichtiefenbegrenzung zu gewährleisten, also auszuschließen, daß möglicherweise auch der Anschlag mit in das betreffende Gewebe eindringt, ist bei einer bevorzugten Ausführungsform der Erfindung vorgesehen, daß der Anschlag im ausgefahrenen Eingriffszustand in radialer Richtung über den Außenkatheter übersteht, wobei der Überstand vorzugsweise ein Mehrfaches des Außendurchmessers des Außenkatheter beträgt. Durch einen derart großen Anschlag kann letztlich sichergestellt werden, daß weder das Werkzeug noch der Außen- oder Innenkatheter das Herzgewebe durchstechen können.

Um die erfindungsgemäße Kathetereinrichtung auch mit einem derart großen Anschlag an die Angriffstelle bringen zu können, weist der Anschlag wenigstens ein im Zuführzustand innerhalb des Außenkatheters angeordnetes, federndes Anschlagmittel auf. Im Zuführzustand befindet sich das Anschlagmittel dann im eingefederten Zustand. Beim Ausfahren des Werkzeuges wird dann auch das Anschlagmittel ausgefahren und öffnet sich nach außen hin in radialer Richtung. Im ausgefederten Zustand verläuft der Anschlag dann vorzugsweise etwa im rechten Winkel zum Außenkatheter.

Als Anschlagmittel kann beispielsweise ein Netz oder eine Spirale vorgesehen sein. Besonders bevorzugt sind eine Mehrzahl von Federschenkeln.

Wenngleich es grundsätzlich möglich ist, den Anschlag bzw. das oder die Anschlagmittel als separate Bauteile auszubilden, ist es von besonderem Vorteil, das Anschlagmittel einstückig mit dem Werkzeug oder dem Innenkatheter auszubilden. In diesem Falle sollte der Innenkatheter dann aus einem Formgedächtnismaterial, insbesondere Teflon bestehen. In gleicher Weise gilt dies für das Werkzeug, das aber auch aus anderen Materialien mit Formgedächtnismaterial bestehen kann.

Von weiterem Vorteil ist es, wenn das Werkzeug aus einem diamagnetischen oder nur geringfügig paramagnetischen Material besteht. Besonders geeignet sind hier Teflon, Nitinol oder ein geeignet fester Polymerkunststoff. Hierdurch ergibt sich letztlich eine MR-Tauglichkeit bzw. -Kompatibilität des erfindungsgemäßen Katheters. Zur Sichtbarmachung des Werkzeugs aus einem derartigen MRT-kompatiblen Material können passive oder aktive Marker oder entsprechende Beschichtungen verwendet werden. Im übrigen versteht es sich, daß die erfindungsgemäße Kathetereinrichtung nicht nur für MRT- sondern auch für CT-Anwendungen geeignet ist. Letztlich läßt es die erfindungsgemäße Ausgestaltung ohne weiteres zu, daß das Werkzeug an der Spitze der Kathetereinrichtung unter MRT-, CT- oder Röntgenkontrolle von einem Einstichort beispielsweise in der Leiste über die Arterien bis in das Herz vorgeführt und dort in die Zielregion eingestochen wird.

Eingangs ist darauf hingewiesen worden, daß es sowohl beim Zuführen des Katheters als auch beim Eingriff in der Zielregion zu Verletzungen kommen kann, wenn die Kathetereinrichtung nicht hinreichend gehandhabt wird. Um die Kathetereinrichtung sowohl beim Zuführen durch die Gefäße als auch beim Eingriff exakt steuern zu können, ist zur Steuerung der Ausrichtung des proximalen Endes des Außenkatheters eine Steuereinrichtung vorgesehen. Durch die Steuerung des proximalen Endes des Außenkatheters kann nicht nur die Zuführung insbesondere an Verzweigungen von Gefäßen verbessert werden, es kann auch das Werkzeug exakt an der vorgesehenen Stelle der Zielregion plaziert werden.

Bei einer besonders einfachen Ausführungsform der Erfindung weist die Steuereinrichtung einen von außen betätigbaren Faden oder Draht auf, der im Bereich des proximalen Endes des Außenkatheters befestigt ist. Durch Zug an dem Faden oder Draht kann das proximale Ende des Außenkatheters sowohl beim Zuführen durch die Gefäße als auch am Zielort von seiner Ausrichtung her gesteuert werden. Durch Zug an dem Draht oder dem Faden tritt nahe des proximalen Endes des Außenkatheters eine Biegung auf. Um dabei in einfacher Weise eine Biegung des Außenkatheters zu erzielen, ist die Wandung des Außenkatheters im Bereich des proximalen Endes an wenigstens einer Stelle verdünnt oder eingekerbt.

Um die Injektion zu erleichterten und das Ausweichen und Abrutschen der Kathetereinrichtung beim Ansetzen an das Gewebe zum Eingriff zu verhindern, ist im Bereich des distalen Endes des Außenkatheters ein abzweigender Anschluß zum Anlegen eines Unterdrucks vorgesehen. Durch den Unterdruck kann sich ein Festsaugen und damit eine Fixierung des Außenkatheters am Gewebe ergeben.

Nachfolgend werden Ausführungsbeispiele der Erfindung anhand der Zeichnung beschrieben. Dabei zeigt
- Fig. 1: eine Ansicht des proximalen Endes einer erfindungsgemäßen Kathetereinrichtung im Zuführzustand,
- Fig. 2: eine der Fig. 1 entsprechende Ansicht einer anderen Ausführungsform einer erfindungsgemäßen Kathetereinrichtung,
- Fig. 3: eine Ansicht der Kathetereinrichtung aus Fig. 2 mit abgebogenem proximalem Ende im Zuführzustand,
- Fig. 4: eine der Fig. 3 entsprechende Ansicht der Kathetereinrichtung aus Fig. 3 mit teilweise ausgefahrener Nadel und
- Fig. 5: eine der Fig. 4 entsprechende Ansicht der Kathetereinrichtung mit ausgefahrener Nadel und ausgefahrenem Anschlag im Injektionszustand.

In den Figuren sind zwei unterschiedliche Ausführungsformen einer Kathetereinrichtung 1 dargestellt, die sich allerdings nur durch die Ausbildung der Biegestelle 10 unterscheiden. Ungeachtet dieses Unterschiedes ist die Kathetereinrichtung 1 für perkutane oder interventionelle Eingriffe wie Injektionen vorgesehen. Allerdings sind auch perkutane Eingriffe anderer Art, beispielsweise Biopsien oder ähnliches möglich. Die Kathetereinrichtung 1 weist einen Außenkatheter 2 und ein Werkzeug für den Eingriff auf. Bei dem Werkzeug handelt es sich vorliegend um eine Injektionsnadel 3, die am proximalen Ende 4 der Kathetereinrichtung 1 vorgesehen ist. Je nach Art des Eingriffs versteht es sich, daß statt einer Injektionsnadel 3 auch eine Biopsiezange, Elektroden oder dergleichen vorgesehen sein können.

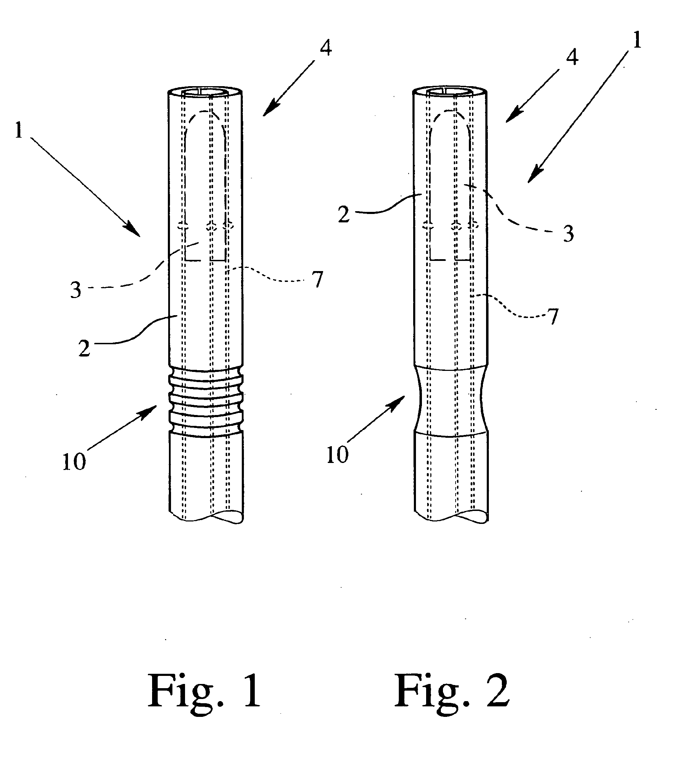

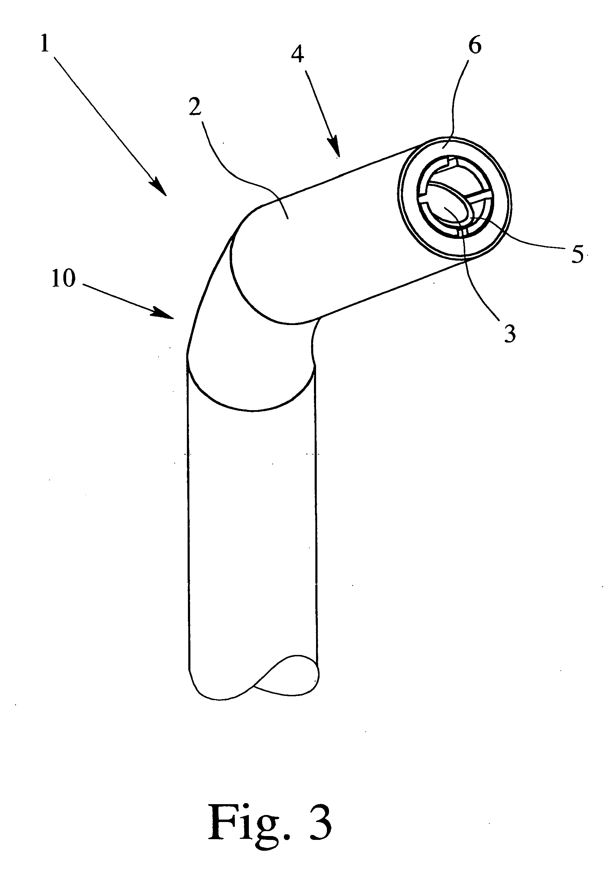

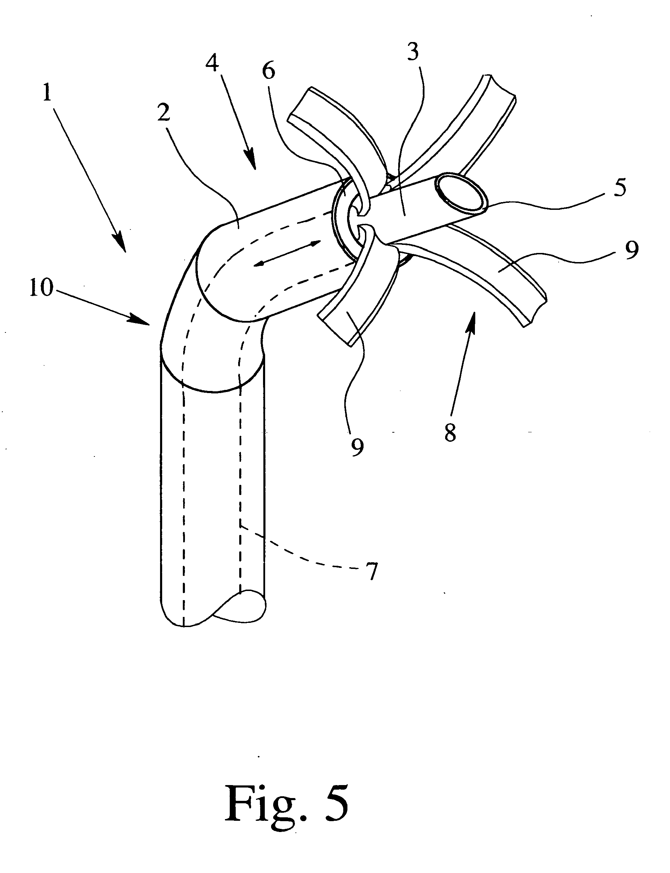

Im in den Figuren 1 bis 3 dargestellten Ausführungsformen befindet sich die Injektionsnadel 3 in einem Zuführzustand, wobei die Injektionsnadel 3 im Außenkatheter 2 aufgenommen ist und die Nadelspitze 5 im Außenkatheter 2 versenkt ist. Dabei steht die Nadelspitze 5 mit ihrem äußersten Ende nicht über die Stirnfläche 6 des Außenkatheters 2 über. Dies ergibt sich insbesondere aus den Fig. 1 bis 3. Die Injektionsnadel 3 ist bei der Kathetereinrichtung 1 relativ zum Außenkatheter 2 bewegbar, nämlich ausfahrbar, so daß zumindest die Nadelspitze 5 im Eingriffszustand, der in Fig. 5 dargestellt ist, über das proximale Ende 4 des Außenkatheters 2 und damit über die Stirnfläche 6 übersteht. Die relative Bewegbarkeit der Injektionsnadel 3 zum Außenkatheter 2 ist dabei im übrigen derart, daß nicht nur ein Ausfahren möglich ist, sondern daß die ausgefahrene Injektionsnadel 3 auch wieder in den Außenkatheter 2 eingefahren werden kann, so daß sich anschließend der in Fig. 3 dargestellte Zustand ergibt.

Zum Ausfahren der Injektionsnadel 3 aus dem Außenkatheter 2 ist ein Innenkatheter 7 vorgesehen, der relativ zum Außenkatheter 2 bewegbar ist. Dabei ist die Injektionsnadel 3 am proximalen Ende des Innenkatheters 7 befestigt. Vorliegend ist die Injektionsnadel 3 mit ihrem hinteren distalen Ende in den Innenkatheter eingesteckt.

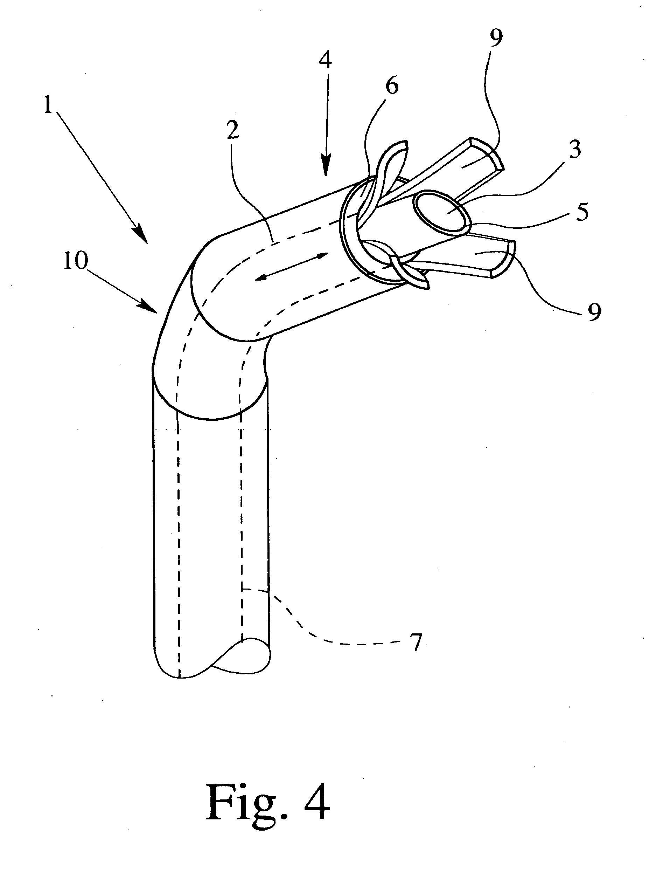

Insbesondere die Fig. 4 und 5 verdeutlichen, daß am Innenkatheter 7 ein Anschlag 8 zur Einstichtiefenbegrenzung der Injektionsnadel 3 bei der Injektion vorgesehen ist. Der Anschlag 8 steht im ausgefahrenen Eingriffszustand, wie dies in Fig. 5 dargestellt ist, in radialer Richtung über den Außenkatheter 2 über. Die radiale Erstreckung des Anschlags 8 ist dabei um ein Mehrfaches größer als der Außendurchmesser des Außenkatheters 2. Der Anschlag 8 weist vorliegend eine Mehrzahl von benachbarten Federschenkeln 9 als Anschlagmittel auf. Die Federschenkel 9 sind einstückig mit dem Innenkatheter 7 ausgebildet. Zur Herstellung der Federschenkel 9 ist der Innenkatheter an seinem proximalen Ende mehrfach eingeschnitten, so daß sich die einzelnen Federschenkel 9 ergeben. Im vorliegenden Fall sind vier Federschenkel 9 vorgesehen, die im ausgefederten Zustand über etwa 90% voneinander beabstandet sind. Den ausgefederten Zustand der Federschenkel zeigt die Fig. 5. Im in den Fig. 1 bis 3 dargestellten Zustand sind die Federschenkel 9 eingefedert und befinden sich im Zuführzustand innerhalb des Außenkatheters 2.

Der Innenkatheter 7 besteht vorliegend wie der Außenkatheter 2 auch aus Teflon, wobei es sich hierbei um ein Formgedächtnismaterial handelt, was für die Ausbildung der Federschenkel 9 des Anschlags 8 mit radialer Erstreckung im ausgefederten Zustand wichtig ist. Die Injektionsnadel 5 besteht vorliegend aus Nitinol, also einem diamagnetischen Material, so daß die Kathetereinrichtung 1 MRT-kompatibel ist. Die Injektionsnadel 5 kann grundsätzlich aber auch aus einem anderen MRT-kompatiblem Material, wie beispielsweise nicht-ferromagnetischem Stahl, einer anderen Metallegierung oder auch aus einem sehr festen Kunststoff oder Kohlefaser bestehen.

Nicht dargestellt ist, daß eine Steuereinrichtung zur Steuerung der Ausrichtung des proximalen Endes 4 des Außenkatheters 2 vorgesehen ist. Die Steuereinrichtung weist einen am proximalen Ende des Außenkatheters 1 befestigten Faden auf, der durch den Außenkatheter 2 nach außen geführt ist. Der Faden läuft dabei parallel zum Innenkatheter 7, aber nicht durch diesen hindurch. Durch Zug an dem Faden ergibt sich eine Biegung des proximalen Endes 4 des Außenkatheters 2 von dem in Fig. 2 dargestellten Zustand zu dem in Fig. 3 dargestellten Zustand. Die Biegung des proximalen Endes 4 wird dadurch begünstigt, daß im Bereich des proximalen Endes 4 die Wandstärke des Außenkatheters 2 verdünnt oder eingekerbt ist. Hierzu sind in den Fig. 1 und 2 mögliche Ausführungsformen zur Herstellung einer Biegestelle 10 vorgesehen.

Eine perkutane Injektion mittels der Kathetereinrichtung 1 erfolgt nun derart, daß die Kathetereinrichtung 1 mit ihrem proximalen Ende von einem Einstichort beispielsweise in der Leiste oder in einer Armbeuge über die Arterien bis in das Herz vorgeführt wird. Dies erfolgt vorzugsweise unter MRT-, CT- oder Röntgenkontrolle. Bei der Bewegung des Katheters innerhalb der Gefäße befindet sich sowohl die Injektionsnadel 3 als auch der Innenkatheter 7 innerhalb des Außenkatheters 2. Die Federschenkel 9 des Anschlags 8 sind eingefedert und umgeben die Nadel 3. Wenn die Kathetereinrichtung 1 bis zum Zielort vorgeführt worden ist, wird der Innenkatheter 7 aus dem Außenkatheter 2 herausgeschoben. Dabei federn dann die Federschenkel 9 des Anschlags 8 nach außen auf. Es findet also eine Entfaltung des Anschlags 8 statt, bis die Injektionsnadel 3 vollständig freigegeben ist, wie dies in Fig. 5 dargestellt ist. Die Injektionsnadel 3 kann dann soweit in das Gewebe eingestochen werden, bis sie durch die am Gewebe anliegenden Federschenkel 9 am weiteren Eindringen gehindert wird. Anschließend kann die Injektion erfolgen. Nach der Injektion wird zunächst der Innenkatheter 7 wieder in den Außenkatheter 2 hineingezogen. Anschließend kann der Außenkatheter 2 herausgezogen werden.

### Bezugszeichenliste:

- 1: Kathetereinrichtung
- 2: Außenkatheter
- 3: Injektionsnadel
- 4: proximales Ende
- 5: Nadelspitze
- 6: Stirnfläche
- 7: Innenkatheter
- 8: Anschlag
- 9: Federschenkel
- 10: Biegestelle

## Patentansprüche

1. Kathetereinrichtung (1) für perkutane Eingriffe, insbesondere für Injektionnen, Biopsien oder dergleichen, mit einem Außenkatheter (2) und einem Werkzeug für den Eingriff, insbesondere einer Injektionsnadel (3), einer Biopsiezange, Elektroden oder dergleichen, am proximalen Ende (4) der Kathetereinrichtung (1),
- wobei das Werkzeug im Zuführzustand im Außenkatheter (2) aufgenommen und die Werkzeugspitze des Werkzeugs im Zuführzustand im Außenkatheter (2) versenkt oder mit dem proximalen Ende des Außenkatheters (2) ausgefluchtet ist,
- wobei das Werkzeug relativ zum Außenkatheter (2) derart bewegbar ist, daß zumindest die Werkzeugspitze im Eingriffszustand über das proximale Ende (4) des Außenkatheters (2) übersteht,
- wobei ein Anschlag (8) zur Eingriffstiefenbegrenzung des Werkzeugs beim Eingriff vorgesehen ist,
- wobei der Anschlag (8) im ausgefahrenen Eingriffszustand in radialer Richtung über den Außenkatheter (2) übersteht,
- wobei der Anschlag (8) wenigstens ein im Zuführzustand innerhalb des Außenkatheters (2) angeordnetes, federndes Anschlagmittel aufweist und, das Anschlagsmittel beim Ausfahren des Werkzeugs ausfedert und sich nach außen hin öffnet und
- wobei das Anschlagmittel als Federschenkel (9) ausgebildet ist,
**dadurch gekennzeichnet,**
**daß** der Anschlag (8) im Eingriffszustand in radialer Richtung eine Erstreckung aufweist, die um ein Mehrfaches größer ist als der Außendurchmesser des Außenkatheters (2),
- wobei die Innenkontur des Anschlagmittels der Außenkontur des Werkzeugs im Bereich der Werkzeugspitze (5) entspricht.

2. Kathetereinrichtung nach Anspruch 1, dadurch gekennzeiehnet, daß zum Ausfahren des Werkzeugs aus dem Außenkatheter (2) ein innerhalb des Außenkatheters (2) relativ zum Außenkatheter (2) bewegbarer Innenkatheter (7) vorgesehen ist, und daß das Werkzeug am proximalen Ende des Innenkatheters (7) vorgesehen ist.

3. Kathetereinrichtung nach Anspruch 1 oder 2, **dadurch gekennzeichnet, daß** das Werkzeug mit seinem distalen Ende an dem Innenkatheter (7) befestigt ist oder daß das Werkzeug einstückig mit dem Innenkatheter (7) ausgebildet ist.

4. Kathetereinrichtung nach einem der vorhergehenden Ansprüche, **dadurch gekennzeichnet, daß** zum Ausfahren des Werkzeugs am proximalen Ende (4) des Außenkatheters (2) eine Vorschubeinrichtung befestigt ist und daß das Werkzeug an der Vorschubeinrichtung befestigt ist.

5. Kathetereinrichtung nach einem der vorhergehenden Ansprüche, **dadurch gekennzeichnet, daß** die Vorschubeinrichtung wenigstens eine Feder aufweist.

6. Kathetereinrichtung nach einem der vorhergehenden Ansprüche, **dadurch gekennzeichnet, daß** wenigstens ein Auslösemittel zur Freigabe der Feder zum Ausfahren des Werkzeugs vorgesehen ist.

7. Kathetereinrichtung nach einem der vorhergehenden Ansprüche, **dadurch gekennzeichnet, daß** zur Freigabe der Feder ein von außen betätigbarer Auslösedraht vorgesehen ist.

8. Kathetereinrichtung nach einem der vorhergehenden Ansprüche, **dadurch gekennzeichnet, daß** der Anschlag am Werkzeug und/oder am Innenkatheter (7) vorgesehen ist.

9. Kathetereinrichtung nach einem der vorhergehenden Ansprüche, **dadurch gekennzeichnet, daß** das Anschlagmittel einstückig mit dem Werkzeug oder dem Innenkatheter (7) ausgebildet ist.

10. Kathetereinrichtung nach einem der vorhergehenden Ansprüche, **dadurch gekennzeichnet daß** der Innenkatheter (7) aus einem Formgedächtnismaterial, insbesondere Teflon, besteht.

11. Kathetereinhchtung nach einem der vorhergehenden Ansprüche, **dadurch gekennzeichnet, daß** das Werkzeug aus einem diamagnetischen oder nur geringfügig paramagnetischen Material, insbesondere Nitinol besteht.

12. Kathetereinrichtung nach einem der vorhergehenden Ansprüche, **dadurch gekennzeichnet, daß** zur Steuerung der Ausrichtung des proximalen Endes (4) des Außenkatheters (2) eine Steuereinrichtung vorgesehen ist.

13. Kathetereinrichtung nach einem der vorhergehenden Ansprüche, **dadurch gekennzeichnet, daß** die Steuereinrichtung einen von außen betätigbaren Faden oder Draht aufweist, der im Bereich des proximalen Endes (4) am Außenkatheter (2) befestigt ist.

14. Kathetereinrichtung nach einem der vorhergehenden Ansprüche, **dadurch gekennzeichnet, daß** die Wandstärke des Außenkatheters (2) zur Bildung einer Biegestelle (10) im Bereich des proximalen Endes (4) verdünnt und/oder einge-kerbt ist.

15. Kathetereinrichtimg nach einem der vorhergehenden Ansprüche, **dadurch gekennzeichnet, daß** im Bereich des distalen Endes des Außenkatheters (2) eine abzweigender Anschluß zum Anlegen eines Unterdrucks vorgesehen ist.

## Claims

1. A catheter device (1) for percutaneous interventions, in particular for injections, biopsies or the like, comprising an outer catheter (2) and a tool for the intervention, in particular an injection needle (3), biopsy forceps, electrodes or the like, at the proximal end (4) of the catheter device (1),
- the tool, in the delivery state, being accommodated in the outer catheter (2) and the tip of the tool, in the delivery state, being recessed in the outer catheter (2) or aligned with the proximal end of the outer catheter (2),
- the tool being movable relative to the outer catheter (2) so that at least the tool tip protrudes beyond the proximal end (4) of the outer catheter (2) in the intervening state,
- a stop (8) being provided to limit the intervening depth of the tool during the intervention,
- the stop (8), in the deployed intervening state, protruding beyond the outer catheter (2) in the radial direction,
- the stop (8) having at least one resilient stop means arranged, in the delivery state, within the outer catheter (2) and the stop means extending and opening outwards upon deployment of the tool, and
- the stop means being designed as spring legs (9),
**characterised in that**,
in the intervening state, the stop (8) has an extent in the radial direction which is several times greater than the outer diameter of the outer catheter (2),
- the inner contour of the stop means corresponding to the outer contour of the tool in the region of the tool tip (5).

2. The catheter device according to claim 1, **characterised in that**, for deployment of the tool from the outer catheter (2), an inner catheter (7) which can be moved within the outer catheter (2) in relation to the outer catheter (2) is provided, and **in that** the tool is provided on the proximal end of the inner catheter (7).

3. The catheter device according to claim 1 or 2, **characterised in that** the tool is fastened by its distal end to the inner catheter (7), or **in that** the tool is formed in one piece with the inner catheter (7).

4. The catheter device according to one of the preceding claims, **characterised in that**, for deployment of the tool, an advancement device is fastened to the proximal end (4) of the outer catheter (2), and **in that** the tool is fastened to the advancement device.

5. The catheter device according to one of the preceding claims, **characterised in that** the advancement device has at least one spring.

6. The catheter device according to one of the preceding claims, **characterised in that** at least one triggering means is provided for releasing the spring to deploy the tool.

7. The catheter device according to one of the preceding claims, **characterised in that** a trigger wire is provided which can be operated externally so as to release the spring.

8. The catheter device according to one of the preceding claims, **characterised in that** the stop is provided on the tool and/or on the inner catheter (7).

9. The catheter device according to one of the preceding claims, **characterised in that** the stop is formed in one piece with the tool or the inner catheter (7).

10. The catheter device according to one of the preceding claims, **characterised in that** the inner catheter (7) consists of a shape-memory material, in particular Teflon.

11. The catheter device according to one of the preceding claims, **characterised in that** the tool consists of a diamagnetic or only slightly paramagnetic material, in particular Nitinol.

12. The catheter device according to one of the preceding claims, **characterised in that** a control device is provided to control alignment of the proximal end (4) of the outer catheter (2).

13. The catheter device according to one of the preceding claims, **characterised in that** the control device has a thread or wire which can be operated externally and which is fastened to the outer catheter (2) in the region of the proximal end (4).

14. The catheter device according to one of the preceding claims, **characterised in that** the wall thickness of the outer catheter (2) is thinned and/or notched to form a bending point (10) in the region of the proximal end (4).

15. The catheter device according to one of the preceding claims, **characterised in that** a branching connection is provided in the region of the distal end of the outer catheter (2) to apply a vacuum.

## Revendications

1. Dispositif de cathéter (1) destiné à des interventions percutanées, en particulier pour des injections, des biopsies ou similaire, comprenant un cathéter extérieur (2) et un outil pour l'intervention, en particulier une aiguille d'injection (3), une pince à biopsie, des électrodes ou similaire, sur l'extrémité proximale (4) du dispositif de cathéter (1),
- dans lequel l'outil est reçu dans le cathéter extérieur (2) dans l'état d'alimentation et la pointe de l'outil est enfoncée dans le cathéter extérieur (2) dans l'état d'alimentation ou est alignée avec l'extrémité proximale du cathéter extérieur (2),
- dans lequel l'outil est mobile par rapport au cathéter extérieur (2) de telle façon qu'au moins la pointe de l'outil fait saillie au-dessus de l'extrémité proximale (4) du cathéter extérieur (2) dans l'état d'intervention,
- dans lequel une butée (8) est prévue pour délimiter la profondeur d'intervention de l'outil lors de l'intervention,
- dans lequel la butée (8) fait saillie en direction radiale au-dessus du cathéter extérieur (2) dans l'état d'intervention déployé,
- dans lequel la butée (8) présente au moins un moyen de butée amortissant disposé à l'intérieur du cathéter extérieur (2) dans l'état d'alimentation et le moyen de butée se détend lors du déploiement de l'outil et s'ouvre vers l'extérieur et
- dans lequel le moyen de butée est formé en tant que branche de ressort (9),
**caractérisé en ce que**
la butée (8) présente une extension en direction radiale dans l'état d'intervention qui est plus grande d'un multiple que le diamètre extérieur du cathéter extérieur (2),
- dans lequel le contour intérieur du moyen de butée correspond au contour extérieur de l'outil au niveau de la zone de la pointe de l'outil (5).

2. Dispositif de cathéter selon la revendication 1, **caractérisé en ce que** pour déployer l'outil hors du cathéter extérieur (2), un cathéter intérieur (7) mobile à l'intérieur du cathéter extérieur (2) par rapport au cathéter extérieur (2) est prévu, et que l'outil est prévu sur l'extrémité proximale du cathéter intérieur (7).

3. Dispositif de cathéter selon la revendication 1 ou 2, **caractérisé en ce que** l'outil est fixé sur le cathéter intérieur (7) par son extrémité distale ou que l'outil est formé de manière monobloc avec le cathéter intérieur (7).

4. Dispositif de cathéter selon l'une des revendications précédentes, **caractérisé en ce qu'**un dispositif d'avancée sur l'extrémité proximale (4) du cathéter extérieur (2) est prévu pour déployer l'outil, et que l'outil est fixé sur le dispositif d'avancée.

5. Dispositif de cathéter selon l'une des revendications précédentes, **caractérisé en ce que** le dispositif d'avancée présente au moins un ressort.

6. Dispositif de cathéter selon l'une des revendications précédentes, **caractérisé en ce qu'**au moins un moyen de déclenchement est prévu pour libérer le ressort pour déployer l'outil.

7. Dispositif de cathéter selon l'une des revendications précédentes, **caractérisé en ce que** pour libérer le ressort, un fil de déclenchement pouvant être actionné par l'extérieur est prévu.

8. Dispositif de cathéter selon l'une des revendications précédentes, **caractérisé en ce que** la butée est prévue sur l'outil et/ou sur le cathéter intérieur (7).

9. Dispositif de cathéter selon l'une des revendications précédentes, **caractérisé en ce que** le moyen de butée est formé de manière monobloc avec l'outil ou le cathéter intérieur (7).

10. Dispositif de cathéter selon l'une des revendications précédentes, **caractérisé en ce que** le cathéter intérieur (7) est en matériau à mémoire de forme, en particulier en Téflon.

11. Dispositif de cathéter selon l'une des revendications précédentes, **caractérisé en ce que** l'outil est en matériau diamagnétique ou seulement légèrement paramagnétique, en particulier en Nitinol.

12. Dispositif de cathéter selon l'une des revendications précédentes, **caractérisé en ce qu'**un dispositif de commande est prévu pour commander l'alignement de l'extrémité proximale (4) du cathéter extérieur (2).

13. Dispositif de cathéter selon l'une des revendications précédentes, **caractérisé en ce que** le dispositif de commande présente un fil ou un câble pouvant être actionné par l'extérieur, lequel est fixé sur le cathéter extérieur (2) au niveau de l'extrémité proximale (4).

14. Dispositif de cathéter selon l'une des revendications précédentes, **caractérisé en ce que** l'épaisseur de paroi du cathéter extérieur (2) est amincie et/ou entaillée au niveau de l'extrémité proximale (4) pour former un point de flexion (10).

15. Dispositif de cathéter selon l'une des revendications précédentes, **caractérisé en ce qu'**un raccordement en dérivation pour appliquer une dépression est prévu au niveau de l'extrémité distale du cathéter extérieur (2).
